Europäisches Patentamt

**(19)** European Patent Office

Office européen des brevets

**(11)** **EP 0 337 296 B1**

**(12)** **EUROPEAN PATENT SPECIFICATION**

**(45)** Date of publication and mention
of the grant of the patent:
**11.12.1996 Bulletin 1996/50**

**(51)** Int. Cl.$^6$: **A61L 15/00**

**(21)** Application number: **89106057.6**

**(22)** Date of filing: **06.04.1989**

**(54)** **A fibrous composition for absorbent pads, a method for the manufacture of an absorbent material from such a composition, and an absorbent material produced by the method**

Faserige Zusammensetzung für Absorptionskissen, Verfahren zur Herstellung eines absorbierenden Materials aus dieser Zusammensetzung und nach diesem Verfahren hergestelltes absorbierendes Material

Composition fibreuse pour tampons absorbants, procédé pour la fabrication d'un matériau absorbant à partir d'une telle composition et matériau absorbant fabriqué selon ce procédé

**(84)** Designated Contracting States:
**AT BE CH DE ES FR GB GR IT LI LU NL SE**

**(30)** Priority: **11.04.1988 IT 6732488**

**(43)** Date of publication of application:
**18.10.1989 Bulletin 1989/42**

**(73)** Proprietor: **ANGELINI RICERCHE S.P.A. -
SOCIETA' CONSORTILE
(or, briefly, "ANGELINI RICERCHE S.P.A.")
00040 Pomezia - Santa Palomba (Roma) (IT)**

**(72)** Inventors:
• **Palumbo, Gianfranco
I-65125 Pescara (IT)**
• **Carlucci, Giovanni
I-66100 Chieti (IT)**
• **D'Ambrosio, Antonio
I-65121 Pescara (IT)**

• **Fors, Staffan
S-791 71 Falun (SE)**
• **Johansson, Birgitta
S-791 91 Falun (SE)**

**(74)** Representative: **Bosotti, Luciano et al
c/o JACOBACCI & PERANI S.p.A.
Corso Regio Parco, 27
10152 Torino (IT)**

**(56)** References cited:
**EP-A- 0 070 164         EP-A- 0 099 428
EP-A- 0 244 486         WO-A-84/03833**

Remarks:
The file contains technical information submitted after the application was filed and not included in this specification

## Description

### Field of the invention

The invention relates to a fibrous composition for absorbent pads, which is made of cellulose fibres and synthetic fibres. The invention also relates to a method for the manufacture of an absorbent material from the composition and to the absorbent article produced by the method.

### Description of the prior art

Disposable sanitary articles, such as, for example, babies' nappies or sanitary towels for women, conventionally have absorbent cores constituted by pads of cellulose fibres produced by the deposition on a porous medium (form) from a flow of cellulose fibres and air. The cellulose fibres are produced by the dry grinding of a sheet of wood pulp, generally conifer wood pulp. This material is cheap and has a good capacity for absorbing body fluids.

In the case of absorbent pads formed with ground wood pulp, it is thought that the liquids are absorbed and retained mainly within the empty spaces which are formed in the network of cellulose fibres, rather than absorbed into individual fibres. The quantity of liquid absorbed by an absorbent body of cellulose fibres is therefore greater the lower its density, that is, the greater its bulk. Consequently, anything which affects the density and can cause the absorbent material to collapse will contribute to a reduction of its absorption capacity.

An absorbent pad which is made of 100% cellulose fibres has low resistance to compression, particularly when wet, that is, the material is compressed considerably when it is subjected to pressure, and the pad does not therefore have a good capacity for retaining liquids under pressure, and thus has a low absorption capacity in use.

The absorption capacity of the absorbent pad can be increased by the addition of water-gelling substances which are generally defined as superabsorbent substances. On the other hand, the introduction of the superabsorbent substances does not completely resolve the problem since the absorbent pad still has little mechanical strength and often breaks in use, preventing the transfer of the liquids to parts which are still dry.

It is known that the strength of an absorbent layer can be increased by its being mixed with certain synthetic fibres. By way of example, the addition of polyolefin fibres to cellulose fibres in order to improve the degree of strength of the absorbent pad, once it has been heat-treated to activate the polyolefin fibres, is known from United States patent No. 4458042.

It is thought that bonding points develop between the cellulose fibres at the moment when the polyolefin fibres melt: the strength of the pad is thus increased.

The polyolefin fibres are very short (they have an average length of approximately 1 mm). This fact presumably increases the chance of the polyolefin fibres acting as a bonding agent between individual cellulose fibres, but the polyolefin fibres completely lose their fibrous characteristics once they are melted. In the finished product, they therefore act as a glue which binds the cellulose fibres together.

An absorbent pad prepared in this manner will have greater strength and a good capacity for absorbing liquids, but a fairly low retention capacity.

In fact, when an absorbent pad soaked with liquid is subjected to pressure, the wet cellulose fibres lose their resilience and the whole pad collapses, regardless of the fact that the cellulose fibres are interconnected to form a framework.

The whole absorbent layer will therefore collapse if subjected to pressure when wet and the liquid will be expelled from the absorbent layer, regardless of the fact that the fibres are bonded together.

It is also known to improve the wettability of synthetic fibres or mixtures of cellulose and synthetic fibres by the treatment of the synthetic fibres with surfactants which develop hydrophilic characteristics in the synthetic fibres.

According to the above-mentioned United States patent No. 4458042, a surfactant selected from a group constituted by non-ionic and ionic surfactants with molecular weights of less than approximately 8000 is used.

The United States patent No. 4578414 proposes, as a surfactant for polyolefin fibres, any one of the following group of materials comprising:

a) an alkylphenol alkoxylate together with or in combination with a mixture of a mono-, a di- and/or a tri-glyceride, or
b) a polyoxyalkylene ester of a fatty acid, or
c) a combination of b) and any compound from a).

For more detailed information concerning these agents, which can make polyolefin fibres hydrophilic, reference is made to the contents of the above-mentioned United States patent specification.

Further, EP-A-0 070 164 describes a fibrous absorbent composition comprising cellulose fibres from wood pulp and conjugate fibres having a heat fusible, lower melting polyethylene sheath and a higher melting polyester core having certain melting properties.

EP-A-0 244 486 describes a water-absorbing material comprising pulp fibres and composite fibres 1 of polyethylene/polypropylene.

WO-A-8 403 833 describes the preparation of tampons from viscose rayon fibres and composite fibres made from polyethylene/polypropylene.

The incorporation of surfactants such as polyoxyethylene alkyl ethers, polyoxyethylene fatty acid esters or glycerin fatty acid esters to improve hydrophilicity of absorbent webs comprising cellulosic fibres and thermoplastic polymer fibres is known - per se - from EP-A-0 099 428.

The object of the invention

The object of the invention is to propose a fibrous composition containing synthetic fibres and cellulose fibres, for the production of an absorbent material, which after heat treatment has good strength of shape even in wet conditions and under pressure.

By means of this invention, as called for in Claim 1, an absorbent material is produced which not only has increased strength of shape, but also an increased capacity for retaining the liquid absorbed (absorption capacity).

The invention also relates to a method for the production of an absorbent body for disposable sanitary products including such an absorbent composition, to the respective absorbent body and to a disposable absorbent article incorporating such an absorbent body.

Summary of the invention

According to the invention, in addition to cellulose fibres, the composition contains a first and a second synthetic polymeric material.

The polymeric materials are constituted by synthetic fibres. Moreover, the melting point of the first polymeric material is lower than that of the second polymeric material .

At least the second polymeric material may be constituted by quite long fibres, which means that at least 90% by weight of the fibres have lengths between 3 and 60 mm, and conveniently lengths between 5 and 20 mm.

The first polymeric material, which has the lower melting point, may be constituted by very short fibres, for example, of an average length of approximately 1 mm, in accordance with United States patent No. 4458042, in order to improve the chance of this fibre fraction to act as a bonding agent in the finished product, or may be constituted by polymeric materials in powder form.

The synthetic fibres are constituted by two-component fibres, in which a first component is constituted by the first polymeric material and the second component consists of the second polymeric material.

In this case, the two-component fibres are longer which means that at least 90% by weight of the two-component fibres have lengths between 3 and 60 mm and preferably lengths between 5 and 20 mm.

In this case, the fineness of the fibres may be between 1 and 10 dtex, preferably between 1.5 and 7 dtex and even more preferably between 1.7 and 4.4 dtex (1 dtex = 0.9 denier).

The two components of the two-component fibres may be arranged side by side or, alternatively, one of the components, consisting of the first polymeric material, may cover the second component which consists of the second polymeric material. In any case, the second component has a higher melting point than the first component.

The second polymeric material conveniently has a melting point at least 15C degrees higher than the first polymeric material.

The two-component fibres are preferably curled.

The fibrous composition contains a percentage of from 2% to 80% by weight of synthetic fibres, calculated on the total weight of the fibrous composition. Preferably, the percentage of synthetic fibres is from 10% to 40% of the total weight of the fibrous mixture.

The first polymeric material may be constituted by any thermoplastic material which has a melting point at least 15C degrees lower than that of the second polymeric material, which may be constituted by polyethylene, polypropylene, copolymers of esters or of esters and other monomers, or copolymers of amides or of amides and other monomers.

One or both of the thermoplastic polymeric materials are treated according to known principles, with one or more surfactants (that is, agents which make the surfaces of the synthetic fibres substantially more hydrophilic), or one or more surfactants may be incorporated in one or in both the thermoplastic polymeric materials.

For example, the surfactants of the type described by United States patent No. 4578414 have been shown to be suitable.

The synthetic fibres and the cellulose fibres may be wet mixed during the normal manufacture of the sheets of cellulose produced from wood pulp and, in this case, the presence of the surfactants may also facilitate the intimate mixing of the synthetic fibres and the cellulose fibres which go to form the densified sheet of synthetic fibres and cellulose fibres in a wholly conventional manner similar to the normal manufacture of cellulose sheets from wood pulp. In this case, the sheet is then dry fiberized according to normal methods known to manufactures of absorbent products.

Alternatively, the cellulose fibres and the synthetic components may be dry mixed, the various fibres (cellulosic and synthetic) being the dry fiberized or reopened according to known techniques and mixed mechanically and/or pneumatically. The presence of the surfactant which impregnates or covers the synthetic fibres has the main function of making the fibres more hydrophilic and therefore more absorbent in the absorbent pad.

In any case, once the fibrous composition constituted by an intimate mixture of cellulose fibres and synthetic fibres has been made, it is dry formed into the shape of the designated article (e.g. an absorbent body for a panty liner, an absorbent body for a sanitary towel, etc.) by pneumatic means according to techniques known to the manufacturers of absorbent products.

It is, however, a characteristic of the invention that whatever the desired shape of the absorbent body of the absorbent article, it is then heated to a temperature higher than the melting point of the first polymer, but lower than the melting point of the second polymer, for a period of time sufficient for the first polymeric material to melt and form bonding points or bridges between at least the higher melting-point synthetic fibres.

Once the absorbent body has cooled, the first synthetic polymeric material sets to link together at least the fibres of the second polymeric material. The fibres of the second polymeric material form a framework of synthetic fibres containing the absorbent material.

This structure has excellent mechanical strength, even when wet, mechanical strength meaning tensile strength, compression strength and resilience.

The good tensile-strength characteristics prevent the absorbent body from breaking in use and therefore avoid the phenomenon of lack of capillary transfer of the liquids to parts of the absorbent body which are still unused.

The good compression strength characteristics mean that the structure yields less under the loads of use and therefore has an improved absorption capacity (the retention of liquids under load).

Finally, the good resilience characteristics, resilience meaning the capacity to regain as much of the undeformed shape as possible after a deforming load has been removed, and therefore to absorb a further quantity of liquid after deformation, means that the absorbent body proposed by the present invention is a definite improvement relative to those of the prior art.

Whilst it is not wished to be bound by particular theories, it would appear that the improvement in absorptive capacity and the resilience is due to the presence of the framework of synthetic fibres which are very resilient both when dry and when wet, unlike the heat-bonded absorbent structures of the prior art, such as that described by United States patent No. 4458042. The bonded fibres seem to constitute a kind of load-bearing structure containing the absorbent cellulose fibres. On the other hand, the structure according to the above-mentioned patent is formed by the same cellulose fibres held together by the fused synthetic material. However, the cellulose fibres are reasonably strong and resilient only when dry and the pseudo-structure therefore collapses under the loads of use, regardless, or almost regardless, of the fact that the cellulose fibres are bonded together.

Detailed description of the invention

The invention will now be described, purely by way of non-limiting example, with reference to the appended drawings, in which:

Fig. 1: shows schematically a first type of two-component fibre,
Fig. 2: shows schematically a second type of two-component fibre,
Fig. 3: shows schematically the structure of the absorbent material according to the invention, and
Fig. 4: is a graph showing a comparison between the wet bulks of absorbent materials in accordance with the present invention and prior-art absorbent materials under different loads.

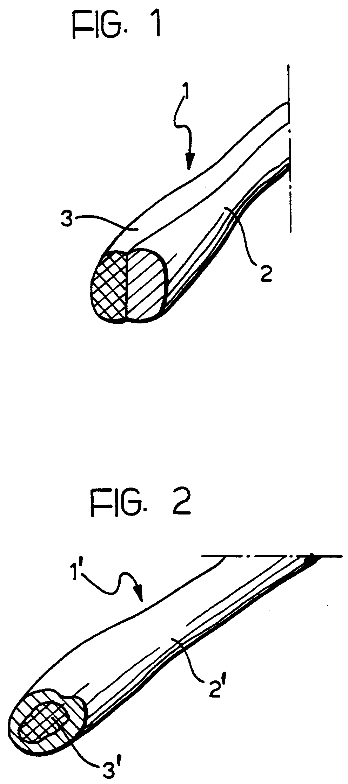

With reference first to Fig. 1, a two-component fibre is generally indicated 1. In accordance with the invention, it is constituted by two components. A first component 2 consists of a first polymeric material, and a second component 3 consists of a second polymeric material. The two components 2 and 3 in this case are arranged side by side.

In this embodiment, the first material 2 consists of polyethylene whilst the second polymeric material 3 consists of polypropylene.

The two components 2 and 3 are thus polyolefins.

The melting point of the PE component is approximately 130°C whilst the PP component has a melting point of approximately 165°C. The ratio of the PE/PP fractions in the two-component fibre is approximately 50/50.

Figure 2 shows an alternative two-component fibre 1'. The outer layer 2' in this case consists of PE of the same type as that used in the previous embodiment whilst the inner material 3' consists of PP, also of the same type as that used in the previous embodiment.

In the tests carried out, the two-component fibres 1 of the PE/PP type were mixed with cellulose fibres of the Stora Fluff type (the trade name of Stora Kopparbergs Bergslag AB).

In one case, the two-component fibres 1 were substantially straight fibres and, in this case, the first polymeric material, polyethylene, contained a first surfactant of the type described in United States patent No. 4578414. Moreover, the fibre was treated with a second surfactant of conventional type, belonging to the sulphosuccinate family.

In another case, a mixture was studied which contained two-component fibres 1 with the same polymeric structure, but in which the fibres had been treated solely with the second type of conventional surfactant. These fibres were curled.

The finenesses of the two-component fibres tested in the compositions given in the tables were in the range 2.2 to 3.3 dtex.

The ratio by weight of the two-component fibres to the cellulose fibres in the mixture was 30/70 in both cases.

In the tests described in Table 1, the two-component fibres were mixed with the cellulose fibres in wet conditions before the drying section of a machine for the continuous production of cellulose pulp from wood. A moist web of fibrous mixture was formed from the mixed paste and was then dried and cut into sheets.

Still with reference to the examples given in Table 1 and Table 2, the cellulose fibres were also mixed with another type of synthetic fibre, known under the trade name HERCULES E338 PULPEX, produced by Hercules Inc., U.S.A. This is constituted by a single-component polyolefin fibre, more precisely, a polyethylene fibre treated in accordance with United States patent No. 4458042.

These fibres were mixed in the same ratio of 30/70 by weight as the two-component fibre 1, and the fibrous composition was formed in the same manner.

Sheets consisting of 100% Stora Fluff were also produced as a reference material.

After dry-fiberizing of the various sheets in a hammer mill, samples were formed from the various fibrous compositions indicated in Table 1 and were tested for their absorption times and their absorptive capacities according to the Scan C 33:80 standard (issued by the Scandinavian Pulp, Paper and Board Testing Committee, Stockholm, Sweden), and for the strength of the pad according to the PFI 1981 method (method for determining the strength of a fibrous pad established by the Norwegian PAPIRINDUSTRIENS FORKNINGSINSTITUTT). All the samples were heat-treated in an oven at the temperatures indicated in Table 2.

The results of the tests described are given in Tables 1 and 2 below.

Table 1

CHARACTERISTICS OF THE SHEETS OF FIBROUS COMPOSITION PRODUCED WET

|  | Composition 1 | Composition 2 | Composition 3 | Composition 4 |
|---|---|---|---|---|
| Weight in grams $(g/m^2)$ | 865 | 866 | 878 | 942 |
| Thickness (mm) | 2.97 | 3.13 | 2.58 | 1.68 |
| Density $(Kg/m^3)$ | 291 | 277 | 340 | 561 |
| Percentage by weight of synthetic fibres (%) | 30 | 30 | 30 | -- |
| Percentage by weight of cellulose fibres (%) | 70 | 70 | 70 | 100 |

Note:

Composition 1: the synthetic fibres are curled, two-component fibres of the type indicated in Fig. 1 and have a PE/PP composition in a proportion of 50/50. They are produced by JACOB HOLM VARDE, Denmark, under the name DANAKLON ES. The fibre is treated with surfactants.

Composition 2: the synthetic fibres are similar two-component fibres but are modified with respect to those of composition 1. In particular, they are straight instead of curled and the PE of the two-component fibre is treated with a surfactant of the type described by US patent No. 4578414. The fibre is also treated with surfactants in this case.

Composition 3: the synthetic fibres are constituted by Pulpex E 338 polyethylene fibres from the American company Hercules Inc.

In all cases, the cellulose fibres were STORA FLUFF fibres of STORA KOPPARBERGS BERGSLAG AB, Sweden.

Table 2

ABSORPTION CHARACTERISTICS OF THE SAMPLES ACCORDING TO THE COMPOSITIONS OF TAB. 1 PRODUCED ACCORDING TO THE SCAN C 33:80 STANDARD, HEAT-TREATED AND SUBJECTED TO A PRESSURE OF 7.5 KPa, AND STRENGTH CHARACTERISTICS ACCORDING TO PFI 1981

| | Composition 1 | | Composition 2 | | Composition 3 | Composition 4 |
|---|---|---|---|---|---|---|
| Heat-treatment temperature ($^o$C) | 135 | 145 | 135 | 145 | 145 | 145 |
| Specific volume when dry (dm$^3$/Kg) | 14.4 | 16.3 | 13.5 | 14.3 | 11.2 | 13.1 |
| Absorption capacity (g/g) | 7.7 | 7.8 | 6.7 | 7.1 | 5.9 | 6.4 |
| Absorption time (s) | 5.4 | 7.2 | 3.1 | 3.4 | 6.4 | 3.2 |
| Strength (N) | >15* | >15* | >15* | >15* | >15* | 6.4 |

* The strength-measuring equipment only determines values below 15N.

Table 1 indicates the characteristics of the sheets of the various fibrous compositions tested.

From the values derived from Table No. 2, it can be seen that the samples formed from the compositions No. 1 and No. 2 according to the present invention have strengths at least equal to those of samples No. 3 formed from cellulose fibres heat-bonded to the Pulpex E338 synthetic fibres but have much greater absorption capacities. Moreover, the samples No. 1 and No. 2 containing two-component fibres also show an improvement in absorption capacity compared

7

with samples No. 4 which are made solely from cellulose fibres, whilst sample No. 3 has a lower absorption capacity than sample No. 4 which is constituted solely by non-heat-bonded cellulose fibres.

Samples No. 1 and No. 2 also show good results as regards their absorption times. In particular, sample No. 2 has absorption times which are entirely comparable with those of sample No. 4, but has a greater absorption capacity.

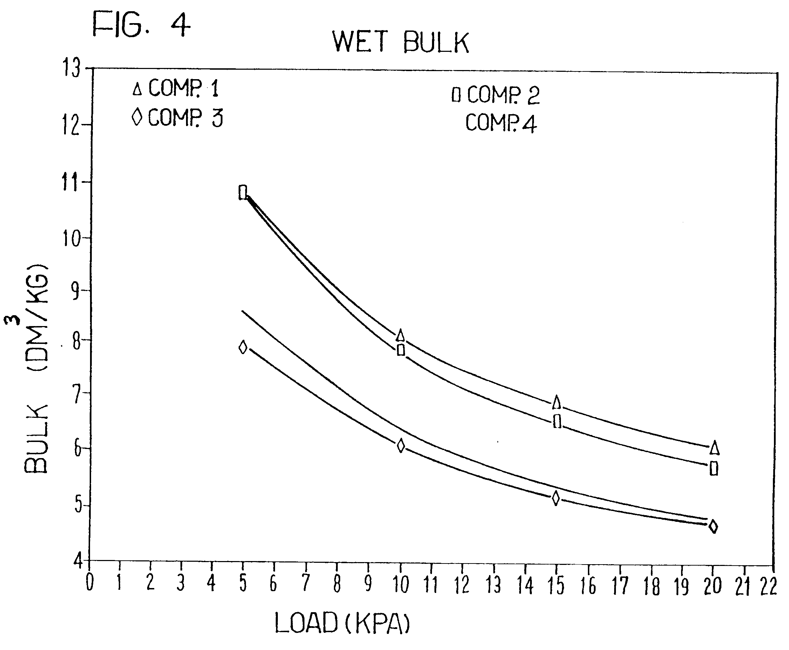

The characteristics of resilience of the pads produced in accordance with the present invention are shown in Table 3 below and in Fig. 4.

Table 3

RESILIENCE CHARACTERISTICS

| | Composition 1 | Composition 2 | Composition 3 | Composition 4 |
|---|---|---|---|---|
| $V_2$: Wet bulk at 20 KPa (dm$^3$/Kg) | 6.1 | 5.8 | 4.7 | 4.8 |
| $V_1$: Wet bulk after removal of load (dm$^3$/Kg) | 12.5 | 12.2 | 8.0 | 8.9 |
| $RI = \dfrac{V_1 - V_2}{V_2} =$ resilience index % | 105 | 110 | 71 | 85 |

The compositions of the samples indicated in Table 3 are the same as those indicated in Table 1, with the difference that the samples are of a different shape and were produced from fibrous compositions obtained by the dry mixing of the individual components of the compositions indicated in Table 1, in the same proportions. Naturally, this falls completely within the spirit and scope of the present invention, which is not limited to the wet mixing of the different individual fibres to produce a sheet of fibrous composition as described above with reference to the samples of Tables No. 1 and No. 2. In fact, it may also be operatively convenient for a producer of absorbent products to start with a normal sheet of cellulose pulp, which is dry-fiberized according to known techniques, and with synthetic fibres (for example in bales) opened up according to known techniques, the synthetic fibres and the cellulose fibres then being dry mixed mechanically and/or pneumatically to produce the fibrous compositions according to the present invention.

The samples of Table No. 3 are constituted by samples with square bases with sides of 38 mm. The sample is dry formed in a square-based sample former operating under vacuum according to known principles similar to those described by the SCAN C:80 standard already mentioned, starting with a dry mixture of cellulose fibres and synthetic fibres intimately mixed beforehand in the ratios indicated in Table 1.

The weight of each individual sample was 2.21 g. Once formed, the samples were subjected to heat-treatment, being heated to temperatures of approximately 135°C.

The samples were then allowed to cool to ambient temperature, and then removed from the square formers. In the example given in Table 3, 100% of the sample No. 4 was constituted by cellulose fibres produced by the dry-fiberizing of a sheet of STORA FLUFF pulp such as that of Table 2, with the difference that in this case the sample was not heat-treated.

Once the square-based samples had been formed as described above, they were completely soaked and then subjected to a cycle of compressions with a dynamometer in the loading range which corresponds to pressures of from 5 to 20 KPa. The dynamometer continuously monitored the depths of the samples and the compression load applied. The speed at which the samples were compressed was 10mm/l'. Figure 4 gives the bulks which correspond to the various loads. Table 3, however, shows the bulks at 20 KPa and the bulks regained by the samples after the removal of the load.

The high bulk values of the samples constituted by the fibrous compositions 1 and 2 according to the present invention can be seen from Table 3. These values are undoubtedly higher than those of the sample No. 4 which is made only of cellulose fibres, whilst the sample No. 3 again has lower absolute values than the sample No. 4 taken as a reference.

If a resilience index RI is defined as:

$$RI = \frac{V_1 - V_2}{V_2} \times 100$$

the data of Table 3 show that the reference sample 4 has an $(RI)_4 = 85\%$, the sample 3 has an $(RI)_3 = 71\%$, whilst the compositions 1 and 2 according to the present invention have RIs > 100%, in particular $(RI)_1 = 105\%$ and $(RI)_2 = 110\%$.

This greater resilience of the fibrous compositions according to the present invention, that is, their greater capacity to regain their undeformed volume once the deforming load has been removed, is a very important characteristic, particularly in consideration of the use for which the heat-stabilised pads according to the present invention are intended. In fact, a disposable sanitary product, such as, for example, a sanitary towel or a baby's nappy, is subject in use to considerable forces which can make a structure made only of cellulose fibres collapse, even if it is heat-bonded. However, an absorbent pad made according to the present invention can resist considerable compressione, whilst retaining a greater quantity of fluid within it (as shown in Table 2 above) and, in particular, can absorb a further quantity of liquid once the load has been removed since it tends to regain its undeformed shape.

It will be appreciated that this particular characteristic of the present invention can prevent the premature loss of the functioning of the structures for absorbing body fluids. For example, in a sanitary towel, this greater resilience means that the absorbent body of the present invention adapts itself better to the anatomy and, moreover, is better at conforming dynamically to anatomical shapes and following the different geometries which occur during the use of a sanitary towel.

Therefore, according to the invention, a heat-bonded structure of cellulose fibres and synthetic fibres is obtained which can appreciably resist compression forces under wet conditions and thus provide improved liquid retention. Moreover, the structure is notably resilient when damp, that is, as described, and, unlike structures which are not heat-bonded, or which are heat-bonded according to the prior art (sample No. 3, Table 3), it can retain a considerable volume once a deforming load has been removed.

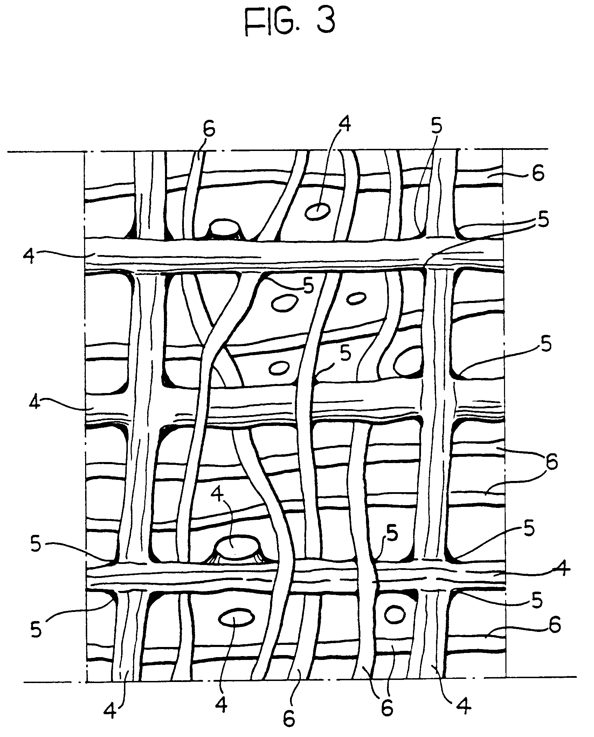

Finally, Fig. 3 shows very schematically the structure of the absorbent material according to the present invention, after heat-treatment. In the drawing, the synthetic fibres are indicated 4. It will be noted that, at the points of contact 5 of adjacent synthetic fibres, the synthetic fibres are joined together so that the low melting-point polymeric component forms bonding points between the fibres. That is, it acts as glue between the various fibres. It should also be noted that the low melting-point polymer 2 (shown in Figure 1) is distributed substantially around the fibres which do not melt.

Since the polymer 2 contains materials which make it wettable, the whole synthetic fibre thus develops hydrophilic characteristics.

The cellulose fibres are indicated 6 in Figure 3. It will be noted that some of these fibres adhere to the synthetic fibres, although most are simply contained within the polymeric framework. With reference to the physical shape of the two-component fibres, the curled fibres develop a more bulky three-dimensional structure.

The graph of Fig. 4 shows the wet bulk of the samples (already described with reference to Table No. 3) when subjected to increasing loads corresponding to pressures of from 5 KPa to 20 KPa. It is noted that the samples of compositions 1 and 2 according to the present invention show decidedly greater wet bulk values than the compositions 3 and 4 throughout the range of loads. On average, the wet bulk of the samples 1 and 2 is more than 20% greater throughout the range of loads. Naturally, the wet bulks of the various samples at 20 KPa are the same as given in Table No. 3.

Therefore, according to the present invention, an absorbent fibrous composition is obtained which, once transformed into an absorbent body for disposable sanitary products, not only has better characteristics of shape, but also a better absorptive capacity in use and better resilience characteristics. It therefore enables absorbent bodies to be produced which are lighter in weight and therefore generally thinner for a given absorptive capacity than those of the prior art, or which, for a given overall weight, enable absorbent bodies of greater absorptive capacity to be produced. In any case, the absorbent bodies produced in accordance with the present invention have better resilience charateristics and are better at adapting to and dynamically following the anatomical shapes of the user.

Naturally, the present invention is not limited to two-component fibres of PE and PP as described, on the contrary, different combinations of polymeric materials are possible, as shown by way of example in Table 4 below.

Table 4

| First polymer | | Second polymer | |
|---|---|---|---|
| Type | Melting point °C | Type | Melting Point °C |
| PE | 130 | PP | 165 |
| PE | 130 | PET | 265 |
| PP | 165 | PET | 265 |
| PE | 130 | PA 6 | 210 |
| PE | 130 | PA 6.6 | 250 |
| PP | 165 | PA 6 | 210 |
| PP | 165 | PA 6.6 | 250 |
| PA 6 | 210 | PET | 265 |
| PA 6.6 | 250 | PET | 265 |
| CoPA | 130-200 | PET | 265 |
| CoPA | 130-200 | PA 6 | 210 |
| CoPA | 130-200 | PA 6.6 | 250 |
| CoPET | 130-210 | PET | 265 |
| CoPET | 130-210 | PA 6.6 | 250 |
| Note: PA=Polyamide PET=Polyester PP=Polypropylene PE=Polyethylene | | | |

## Claims

1. An absorbent fibrous composition comprising a mixture of cellulose fibers and at least first and second polymeric materials wherein the first (2,2') and second (3,3') polymeric materials are present as a two component synthetic fiber, wherein the at least first and second polymeric materials are arranged side by side in the two component synthetic fiber or wherein the first polymeric material is arranged around the second polymeric material as an enclosing sheath, the first and second polymeric materials comprising from 2 to 80%, and preferably 10 to 40% by weight of the fibrous composition, wherein at least one of the first and second polymeric materials comprises a surfactant which renders the polymeric material permanently hydrophilic, the first polymeric material having a melting point at least 15°C lower than that of the second polymeric material, and wherein the absorbent fibrous composition is heated to a temperature higher than the melting point of the first polymeric material but lower than the melting point of the second fibrous material for a period of time sufficient for the first polymeric material to melt and form bonding points between the two component synthetic fibers present and yield a polymeric framework which is a load-bearing structure which contains the majority of the cellulose fibers contained within the polymeric framework but not bonded to the at least first and second polymeric materials, which absorbent fibrous composition exhibits good shape retention under wet conditions and under pressure, wherein the at least first and second polymeric materials are selected from combinations from the group consisting of polyethylene and polypropylene; polyethylene and polyester; polypropylene and polyester; polyethylene and polyamide; polypropylene and polyamide; and polyamide and polyester.

2. An absorbent fibrous composition according to claim 1, wherein at least 90% by weight of the synthetic fibres of the second polymeric material have lengths of between 3 and 60mm.

3. An absorbent fibrous composition according to claim 1 or claim 2, wherein at least 90% by weight of the synthetic fibres of the second polymeric material have lengths of between 5 and 20mm.

4. An absorbent fibrous composition according to any one of claims 1 to 3, wherein the synthetic fibres have a fineness of between 1 and 10 dtex.

5. An absorbent fibrous composition according to any one of claims 1 to 3, wherein the synthetic fibres have a fineness of between 1.5 and 7 dtex.

6. An absorbent fibrous composition according to any one of claims 1 to 3, wherein the synthetic fibres have a fineness of between 1.7 and 4.4 dtex.

7. An absorbent fibrous composition according to any one of claims 1 to 6, wherein the two components of the two-component fibre are arranged side by side.

8. An absorbent fibrous composition according to any one of claims 1 to 6 wherein the first component is arranged around the second component as an enclosing sheath.

9. An absorbent fibrous composition according to any one of claims 1 to 8, wherein the two-component fibres, are curled.

10. An absorbent fibrous composition according to any one of claims 1 to 9, wherein the surfactant consists of at least one of the following agents:

(a) an alkylphenol alkoxylate together with or in combination with a mixture of a mono-, a di-, and/or a tri-glyceride, or
(b) a polyoxyalkylene ester of a fatty acid, or
(c) a combination of (b) and any compound from (a).

11. An absorbent fibrous composition according to any one of claims 1 to 10, wherein the cellulose fibres are derived from chemical wood pulp, chemically modified thermomechanical wood pulp, thermomechanical wood pulp, mechanical wood pulp, or a mixture of two or more of these pulps.

12. An absorbent fibrous composition according to any one of claims 1 to 11, wherein the composition is produced by the wet mixing of the cellulose fibres and the polymeric materials and is in dried-sheet form.

13. An absorbent fibrous composition according to any one of claims 1 to 11, wherein the composition is produced by the dry mixing of the cellulose fibres and the polymeric materials.

14. A method for the production of an absorbent body for disposable sanitary products, constituted by an absorbent fibrous composition according to any one of claims 1 to 13, comprising the following steps:

- the forming of the fibrous composition into an absorbent body of the desired shape,
- the heating of the absorbent body to a temperature at least equal to the melting point of the first polymeric material but below the melting point of the second polymeric material for a period of time such as to melt the first polymeric material and develop bonding points or bridges between at least the synthetic fibres which have not melted,

- the cooling of the absorbent body to a temperature such that the first polymeric material resolidifies and the bonding points or bridges bond together at least the synthetic fibres to form a framework constituted by synthetic fibres containing the cellulose fibres.

15. An absorbent body for disposable sanitary products, including the absorbent fibrous composition of any of claims 1 to 13.

16. An absorbent body according to claim 15, wherein at least some of the cellulose fibres contained in the framework are bonded to the framework by means of the first polymeric material.

17. A disposable absorbent article incorporating an absorbent body according to claim 15 or 16.

**Patentansprüche**

1. Eine absorbierende faserige Zusammensetzung, die eine Mischung aus Zellulosefasern und mindestens ersten und zweiten polymeren Materialien umfaßt, wobei die ersten und zweiten polymeren Materialien als aus zwei Komponenten bestehende Kunststoffaser vorhanden sind, wobei die mindestens ersten und zweiten polymeren Materialien in der Zweikomponenten-Kunststoffaser Seite an Seite angeordnet sind, oder wobei das erste polymere Material als umschließende Hülle um das zweite polymere Material angeordnet ist, wobei die ersten und zweiten polymeren Materialien von 2 bis 80 und vorzugsweise 10 bis 40 Gewichtsprozent der faserigen Zusammensetzung bilden, wobei mindestens eines der ersten und zweiten polymeren Materialien einen grenzflächenaktiven Stoff aufweist, welcher das polymere Material dauerhaft wasseranziehend macht, wobei das erste polymere Material einen Schmelzpunkt hat, der mindestens 15 °C unter dem des zweiten polymeren Materials liegt und wobei die absorbierende faserige Zusammensetzung auf eine Temperatur erhitzt wird, die höher ist als der Schmelzpunkt des ersten polymeren Materials aber niedriger als der Schmelzpunkt des zweiten polymeren Materials, für eine Zeitspanne, die ausreicht, um das erste polymere Material zu schmelzen und Verbindungspunkte zwischen den vorhandenen aus zwei Komponenten bestehenden Kunststofffasern zu bilden und ein polymeres Rahmentragwerk hervorzubringen, welches eine tragende Struktur ist, die den Großteil der Zellulosefasern enthält, die im polymeren Rahmentragwerk enthalten aber nicht mit den mindestens ersten und zweiten polymeren Materialien verbunden sind, wobei die absorbierende faserige Zusammensetzung bei Feuchtigkeit und unter Druck gute Formbeständigkeit zeigt, wobei die mindestens ersten und zweiten polymeren Materialien aus Kombinationen aus der Gruppe ausgewählt sind, die aus Polyethylen und Polypropylen; Polyethylen und Polyester; Polypropylen und Polyester; Polyethylen und Polyamid; Polypropylen und Polyamid; und Polyamid und Polyester besteht.

2. Eine absorbierende faserige Zusammensetzung nach Anspruch 1, wobei mindestens 90 Gewichtsprozent der Kunststoffasern des zweiten polymeren Materials Längen von zwischen 3 und 60 mm haben.

3. Eine absorbierende faserige Zusammensetzung nach Anspruch 1 oder 2, wobei mindestens 90 Gewichtsprozent der Kunststoffasern des zweiten polymeren Materials Längen zwischen 5 und 20 mm haben.

4. Eine absorbierende faserige Zusammensetzung nach einem der Ansprüche 1 bis 3, wobei die Kunststoffasern eine Feinheit von zwischen 1 und 10 dtex haben.

5. Eine absorbierende faserige Zusammensetzung nach einem der Ansprüche 1 bis 3, wobei die Kunststoffasern eine Feinheit von zwischen 1.5 und 7 dtex haben.

6. Eine absorbierende faserige Zusammenfassung nach einem der Ansprüche 1 bis 3, wobei die Kunststoffasern eine Feinheit von zwischen 1,7 und 4,4 dtex haben.

7. Eine absorbierende faserige Zusammensetzung nach einem der Ansprüche 1 bis 6, wobei die zwei Komponenten der Zweikomponentenfaser nebeneinander angeordnet sind.

8. Eine absorbierende faserige Zusammensetzung nach einem der Ansprüche 1 bis 6, wobei die erste Komponente um die zweite Komponente als schließende Hülle angeordnet ist.

9. Eine absorbierende faserige Zusammensetzung nach einem der Ansprüche 1 bis 8, wobei die Zweikomponentenfasern gekräuselt sind.

**10.** Eine absorbierende faserige Zusammensetzung nach einem der Ansprüche 1 bis 9, wobei der grenzflächenaktive Stoff aus mindestens einem der folgenden Mittel besteht:

a) einem Alkylphenol Alkoxilat zusammen mit oder in Kombination mit einer Mischung eines Mono-, eines Di- und/oder eines Triglyzerides oder

b) einem Polyoxyalkylenester einer Fettsäure oder

c) einer Kombination von (b) und irgendeiner Zusammensetzung aus (a).

**11.** Eine absorbierende faserige Zusammenfsetzung nach einem der Ansprüche 1 bis 10, wobei die Zellulosefasern aus chemischer Holzpulpe, chemisch modifizierter thermomechanischer Holzpulpe, thermomechanischer Holzpulpe, mechanischer Holzpulpe oder einer Mischung aus zwei oder mehreren dieser Pulpen stammen.

**12.** Eine absorbierende faserige Zusammensetzung nach einem der Ansprüche 1 bis 11, wobei die Zusammensetzung durch das nasse Mischen der Zellulosefasern und der polymeren Materialien erzeugt wird und in der Form getrockneter Platten ist.

**13.** Eine absorbierende faserige Zusammensetzung nach einem der Ansprüche 1 bis 11, wobei die Zusammenfassung durch das trockene Mischen der Zellulosefasern und der polymeren Materialien erzeugt wird.

**14.** Ein Verfahren zur Herstellung eines absorbierenden Körpers für Einweghygieneartikel, der von einer absorbierenden faserigen Zusammensetzung nach einem der Ansprüche 1 bis 13 gebildet wird mit den folgenden Verfahrensschritten:

- das Formen der faserigen Zusammensetzung zu einem absorbierenden Köprer der gewünschten Form,

- das Erhitzen des absortierenden Körpers mindestens auf eine Temperatur gleich dem Schmelzpunkt des ersten polymeren Materials aber niedriger als der Schmelzpunkt des zweiten polymeren Materials über einen Zeitabschnitt, um derart das erste polymere Material zu schmelzen und Verbindungspunkte oder Brücken zwischen mindestens den Kunststoffasern, die nicht geschmolzen wurden, zu bilden.

- das Kühlen des absorbierenden Körpers auf eine Temperatur, bei der das erste polymere Material wieder erstarrt und die Verbindungspunkte oder Brücken mindestens die Kunststoffasern verbinden, um ein Rahmentragwerk zu bilden, das von Kunststoffasern gebildet wird und die Zellulosefasern enthält.

**15.** Ein absorbierender Körper für Einweghygieneartikel, die die absorbierende faserige Zusammensetzung nach einem der Ansprüche 1 bis 13 enthalten.

**16.** Ein absorbierender Körper nach Anspruch 15, wobei mindestens einige der Zellulosefasern, die im Rahmentragwerk enthalten sind, mit dem Rahmentragwerk durch das erste polymere Material verbunden sind.

**17.** Ein absorbierender Einwegartikel, der einen absorbierenden Körper nach Anspruch 15 oder 16 enthält.

## Revendications

**1.** Composition fibreuse absorbante comprenant un mélange de fibres de cellulose et d'au moins un premier et un second matériaux polymères où les premier (2, 2') et second (3, 3') matériaux polymères sont présents en tant que fibre synthétique à deux composants, dans laquelle lesdits au moins premier et second matériaux polymères sont disposés côte à côte dans la fibre synthétique à deux composants ou dans laquelle le premier matériau polymère est disposé autour du second matériau polymère en tant que gaine enveloppante, les premier et second matériaux polymères constituant de 2 à 80 %, et, de préférence, de 10 à 40 % en poids de la composition fibreuse, dans laquelle au moins un des premier et second matériaux polymères comprend un agent tensio-actif qui rend le matériau polymère hydrophile en permanence, le premier matériau polymère ayant un point de fusion inférieur d'au moins 15° à celui du second matériau polymère, et dans laquelle la composition fibreuse absorbante est chauffée à une température supérieure au point de fusion du premier matériau polymère mais inférieure au point de fusion du second matériau fibreux pendant une durée suffisante pour que le premier matériau polymère fonde et forme des points de liaison entre les fibres synthétiques à deux composants présentes et produise une ossature polymère qui est une ossature porteuse qui renferme la majorité des fibres de cellulose contenues au sein de l'ossature polymère mais non liée auxdits au moins premier et second matériaux polymères, laquelle composition fibreuse absorbante a une conservation de forme satisfaisante dans des conditions d'humidité et sous pression, dans laquelle lesdits au moins premier et second matériaux polymères sont sélectionnés parmi des combinaisons du groupe consistant en polyéthylène et polypropylène; polyéthylène et polyester; polypropylène et polyester; polyéthylène et polyamide; polypropylène et polyamide; et polyamide et polyester.

2. Composition fibreuse absorbante selon la revendication 1, dans laquelle au moins 90 % en poids des fibres synthétiques du second matériau polymère ont une longueur comprise entre 3 et 60 mm.

3. Composition fibreuse absorbante selon la revendication 1 ou la revendication 2, dans laquelle au moins 90 % en poids des fibres synthétiques du second matériau polymère ont une longueur comprise entre 5 et 20 mm.

4. Composition fibreuse absorbante selon l'une quelconque des revendications 1 à 3, dans laquelle les fibres synthétiques ont une grosseur comprise entre 1 et 10 dtex.

5. Composition fibreuse absorbante selon l'une quelconque des revendications 1 à 3, dans laquelle les fibres synthétiques ont une grosseur comprise entre 1,5 et 7 dtex.

6. Composition fibreuse absorbante selon l'une quelconque des revendications 1 à 3, dans laquelle les fibres synthétiques ont une grosseur comprise entre 1,7 et 4,4 dtex.

7. Composition fibreuse absorbante selon l'une quelconque des revendications 1 à 6, dans laquelle les deux composants de la fibre à deux composants sont disposés côte à côte.

8. Composition fibreuse absorbante selon l'une quelconque des revendications 1 à 6, dans laquelle le premier composant est disposé autour du second composant en tant que gaine enveloppante.

9. Composition fibreuse absorbante selon l'une quelconque des revendications 1 à 8, dans laquelle les fibres à deux composants sont ondulées.

10. Composition fibreuse absorbante selon l'une quelconque des revendications 1 à 9, dans laquelle l'agent tensio-actif consiste en au moins un des agents suivants :

(a) alkylphénol alkoxylate avec ou en combinaison avec un mélange de mono, di- et/ou tri-glycéride, ou
(b) polyoxyalkylène ester d'acide gras, ou
(c) une combinaison de (b) et d'un composé de (a).

11. Composition fibreuse absorbante selon l'une quelconque des revendications 1 à 10, dans laquelle les fibres de cellulose sont dérivées de cellulose chimique, de cellulose thermomécanique modifiée chimiquement, de cellulose thermomécanique, de cellulose mécanique, ou d'un mélange de deux ou plusieurs de ces celluloses.

12. Composition fibreuse absorbante selon l'une quelconque des revendications 1 à 11, dans laquelle la composition est produite par le mélange par voie humide des fibres de cellulose et des matériaux polymères puis est en forme de feuille séchée.

13. Composition fibreuse absorbante selon l'une quelconque des revendications 1 à 11, dans laquelle la composition est produite par le mélange par voie sèche des fibres de cellulose et des matériaux polymères.

14. Procédé pour la fabrication d'un corps absorbant pour des produits hygiéniques jetables, constitué par une composition fibreuse absorbante selon l'une quelconque des revendications 1 à 13, comprenant les phases suivantes :

- la formation de la composition fibreuse en un corps absorbant de la forme souhaitée,
- le chauffage du corps absorbant à une température au moins égale au point de fusion du premier matériau polymère mais inférieure au point de fusion du second matériau polymère pendant une durée permettant de faire fondre le premier matériau polymère et créer des points ou ponts de liaison entre au moins les fibres synthétiques qui n'ont pas fondues,
- le refroidissement du corps absorbant à une température telle que le premier matériau polymère se solidifie de nouveau et les points ou ponts de liaison lient ensemble au moins les fibres synthétiques pour former une ossature constituée par les fibres synthétiques contenant les fibres de cellulose.

**15.** Corps absorbant pour produits hygiéniques jetables, comprenant la composition fibreuse absorbante selon l'une quelconque des revendications 1 à 13.

**16.** Corps absorbant selon la revendication 15, dans lequel au moins certaines des fibres de cellulose contenues dans l'ossature sont liées à l'ossature au moyen du premier matériau polymère.

**17.** Article absorbant jetable comprenant un corps absorbant selon la revendication 15 ou 16.

## FIG. 1

## FIG. 2

# FIG. 3

FIG. 4

WET BULK

△ COMP. 1    □ COMP. 2
◇ COMP. 3    COMP. 4

BULK (DM/KG)

LOAD (KPA)